# EUROPEAN PATENT APPLICATION

(11) **EP 4 810 467 A1**
(43) Date of publication of application: **23.09.2026**
(21) Application number: 25863878.2
(22) Date of filing: 29.07.2025
(51) Int. Cl.: C07C 29/76, C07C 29/04, C07C 31/10

(54) **METHOD FOR PREPARING ISOPROPYL ALCOHOL**

(30) Priority: 07.09.2024 KR 20240121956; 25.07.2025 KR 20250101524
(71) Applicant: LG Chem, Ltd., Seoul 07336 (KR)
(72) Inventor: OH, Jai June, Seoul 07796 (KR); KIM, Hyun Ju, Seoul 07796 (KR); LEE, Sung Kyu, Seoul 07796 (KR); RYU, Jin Sook, Seoul 07796 (KR); HWANG, Sung June, Seoul 07796 (KR); JANG, Kyung Soo, Seoul 07796 (KR)
(74) Representative: Goddar, Heinz J.
(86) International application number: PCT/KR2025/011228
(87) International publication number: WO 2026/054328

(57) **Abstract**

Provided is a method for preparing isopropyl alcohol including: supplying a propylene monomer and reaction water to a reactor as a feed stream to produce a gaseous reaction product including isopropyl alcohol, purifying isopropyl alcohol from the gaseous reaction product and recovering process water, and passing the process water through an arsenic removal device including a first filtration layer, adsorbent layer, and a second filtration layer.

## Description

### [Technical Field]

### CROSS-REFERENCE TO RELATED APPLICATIONS

The present application claims the benefit of priorities to Korean Patent Application No. 10-2024-0121956, filed on September 7, 2024, and Korean Patent Application No. 10-2025-0101524, filed on July 25, 2025, the entire contents of which are incorporated herein as a part of the specification.

### Technical Field

The following disclosure relates to a method for preparing isopropyl alcohol, and more particularly, to a method for preparing high-quality isopropyl alcohol by removing arsenic included in process water circulating in the process.

### [Background Art]

Isopropyl alcohol (IPA) is used for various purposes including a solvent for cleaning agents, raw materials for industrial paints and reagents, paints, inks, and the like in the electronics industry such as manufacture of semiconductor or liquid crystal display (LCD).

Isopropyl alcohol may be prepared by reacting a propylene monomer and water. For example, a propylene monomer and water are reacted in a gas phase in a reaction part to obtain a reaction product including isopropyl alcohol, an unreacted propylene monomer, unreacted water, and by-products, and the gaseous reaction product passes through a rear stage process tower to separate the propylene monomer, water, and the by-product, thereby purifying isopropyl alcohol.

In this process, water used as the reaction water is heated and then supplied to a reactor to produce isopropyl alcohol by a reaction with the propylene monomer, and unreacted water may be separated in an isopropyl alcohol purification part, cooled, recycled as process water to be used for washing an absorption tower and an organic material (hydrocarbons) removal tower, and partly discharged as wastewater. As such, water added during the process of preparing isopropyl alcohol is continuously circulated as process water, and the process water may include a small amount of arsenic.

Since arsenic causes degradation of IPA quality, it needs to be removed. In general, a process of removing impurities by a method such as distillation is being performed in an IPA purification part, but since arsenic is decomposed or sublimated at or below an operation temperature of a distillation tower of the purification part of IPA, it is difficult to completely remove arsenic by a distillation method, due to the phenomenon of vaporization into a gas phase. Thus, in order to prepare high-quality isopropyl alcohol, the development of technology of effectively removing arsenic circulating in the process is needed.

### [Disclosure]

### [Technical Problem]

An object of the present disclosure is to provide a method for preparing high-quality isopropyl alcohol by continuously removing arsenic in process water in a process of preparing isopropyl alcohol.

However, the object to be solved in the present disclosure is not limited to the object mentioned above, and other objects which are not mentioned may be clearly understood by a person skilled in the art from the following descriptions.

### [Technical Solution]

In one general aspect, a method for preparing isopropyl alcohol includes: supplying a propylene monomer and reaction water to a reactor as a feed stream to produce a gaseous reaction product including isopropyl alcohol; purifying isopropyl alcohol from the gaseous reaction product and recovering process water; and passing the process water through an arsenic removal device including a first filtration layer, an adsorbent layer, and a second filtration layer.

In addition, the passing of process water through an arsenic removal device including a first filtration layer, an adsorbent layer, and a second filtration layer may include passing the process water through the first filtration layer, the adsorbent layer, and the second filtration layer inside the arsenic removal device sequentially.

### [Advantageous Effects]

The method for preparing isopropyl alcohol of the present disclosure may produce high-quality IPA by continuously removing arsenic in the process water through adsorption to prevent circulation of arsenic in the process.

An effect which may be obtained in the present disclosure is not limited to the effects mentioned above, and other effects which are not mentioned will be understood clearly by a person with ordinary skill in the art to which the present disclosure pertains from the following description.

### [Description of Drawings]

FIG. 1 is a process diagram showing an entire process flow of the method for preparing isopropyl alcohol according to an exemplary embodiment of the present disclosure.
FIG. 2 is a process diagram which embodies the process A of FIG. 1
FIG. 3 shows a structure of an arsenic removal device used in the method for preparing isopropyl alcohol according to an exemplary embodiment of the present disclosure.
FIG. 4 shows a structure of an arsenic removal device used in the method for preparing isopropyl alcohol according to an exemplary embodiment of the present disclosure.
FIG. 5 shows a structure of an arsenic removal device used in the method for preparing isopropyl alcohol according to an exemplary embodiment of the present disclosure.
FIG. 6 shows an arsenic removal rate depending on an empty bed contact time in an exemplary embodiment of the present disclosure.
FIG. 7 is a graph comparing the number of days it took for an arsenic concentration in a product to reach 100% from 4,000% depending on an arsenic removal rate in circulating water, as compared with the management standard.

### [Best Mode]

The terms and words used in the description and claims of the present disclosure are not to be construed limitedly as having general or dictionary meanings but are to be construed as having meanings and concepts meeting the technical ideas of the present disclosure, based on a principle that the inventors are able to appropriately define the concepts of terms in order to describe their own disclosure in the best mode.

Regarding description of the drawings, similar reference numerals may be used for similar or related constituent elements.

A singular form of a noun corresponding to an item may include one or a plurality of items, unless otherwise explicitly stated in the relevant context.

In the present disclosure, each of the phrases such as "A or B", "at least one or A and B", "at least one of A or B", "A, B, or C", "at least one of A, B, and C", and "at least one of A, B, or C" may include any one of the items listed together with the corresponding phrase of the phrases or all possible combinations of them.

The term "and/or" includes a combination of a plurality of described related constituent elements or any one of a plurality of described related constituent elements.

The terms such as "1st" or "2nd", or "first" or "second" may be simply used for distinguishing a corresponding constituent element from other corresponding constituent elements, and the corresponding constituent elements are not limited in other aspects (e.g.: importance or order).

In addition, the terms such as "front surface", "back surface", "upper surface", "lower surface", "side surface", "left", "right", "upper", and "lower" used in the present disclosure are defined based on drawings, and the shape and position of each constituent element are not limited by the terms.

The terms such as "comprises" or "have" are intended to specify the presence of stated features, steps, operations, constituent elements, parts, or a combination thereof, but do not preclude the presence or addition of one or more other features, numerals, steps, operations, constituent elements, parts, or a combination thereof.

When it is described that a constituent element is "connected to", "combined with", "supported on", or "in contact with" other constituent elements, it includes not only the case in which the constituent elements are directly connected, combined, supported, or in contact, but also the case in which they are indirectly connected, combined, supported, or in contact through a third constituent element.

When it is described that a constituent element is positioned "on" other constituent element, it includes not only the case in which the constituent element is in contact with another constituent element, but also the case in which another constituent element is present between the two constituent elements.

In addition, when unique manufacture and material tolerances are suggested in the mentioned meaning, the terms such as "about" and "substantially" used in the present disclosure are used in the meaning of the numerical value or in the meaning close to the numerical value, and are used for preventing the disclosure mentioning a correct or absolute numerical value for better understanding of the present disclosure from being unfairly used by an unconscionable infringer.

The term "stream" used in the present disclosure may refer to a fluid flow in a process or may refer to a fluid itself flowing in a pipe. Specifically, the stream may refer to both a fluid itself flowing in a pipe connecting each device and a fluid flow. In addition, the fluid may include any one or more components of gas, liquid, and solid.

The term "upper portion" used in the present disclosure refers to, unless otherwise stated, a point at 0% to 20% of the height measured downward from the top of a device, and specifically, may refer to a top (tower top). In addition, the term "lower portion" refers to a point at 80% to 100% of the height measured downward from the top of a device, and specifically, may refer to a bottom (tower bottom).

In addition, "pressure" mentioned in the present disclosure refers to a gauge pressure measured based on atmospheric pressure.

The term "empty bed contact time (EBCT)" used in the present disclosure refers to a time during which a continuously flowing fluid substantially resides in a device or a specific area when passing the fluid through the device or the specific area and is calculated by "(volume of device) / (volume flow rate of fluid passing through device)" or "(volume of specific area) / (volume flow rate of fluid passing through specific area)", and the unit is hour (h). For example, when a fluid of 0.5 m³/h continuously flows into a device having a volume of 1 m³, the empty bed contact time is (1/0.5)=2h, which means that the fluid residing in the device for 2 hours.

When catalysts exchange in the process of preparing IPA, arsenic is circulated in the process for the reason of arsenic outflow in a carrier and the like, and thus, arsenic is eluted in the IPA product. Specifically, though IPA is produced by a gaseous reaction, arsenic is partly decomposed or sublimated in a distillation tower, so that arsenic is detected in a gas phase, and it is difficult to completely remove arsenic by a distillation method. Thus, the process water is circulated in a state of containing arsenic, and arsenic is detected in the product for a long time, resulting in quality deterioration.

Thus, in the present disclosure, arsenic present in arsenic-containing circulating water and IPA products is intended to be removed. More specifically, high-quality isopropyl alcohol is intended to be prepared by preventing circulation of arsenic in the process by continuously removing arsenic in process water including the highest content of arsenic by adsorption.

The method for preparing isopropyl alcohol according to an exemplary embodiment of the present disclosure includes: (S1) supplying a propylene monomer and reaction water to a reactor as a feed stream to produce a gaseous reaction product including isopropyl alcohol; (S2) purifying isopropyl alcohol from the gaseous reaction product and recovering process water; and (S3) passing the process water through an arsenic removal device including a first filtration layer, an adsorbent layer, and a second filtration layer. In addition, (S3) may include passing the process water through the first filtration layer, the adsorbent layer, and the second filtration layer inside the arsenic removal device sequentially.

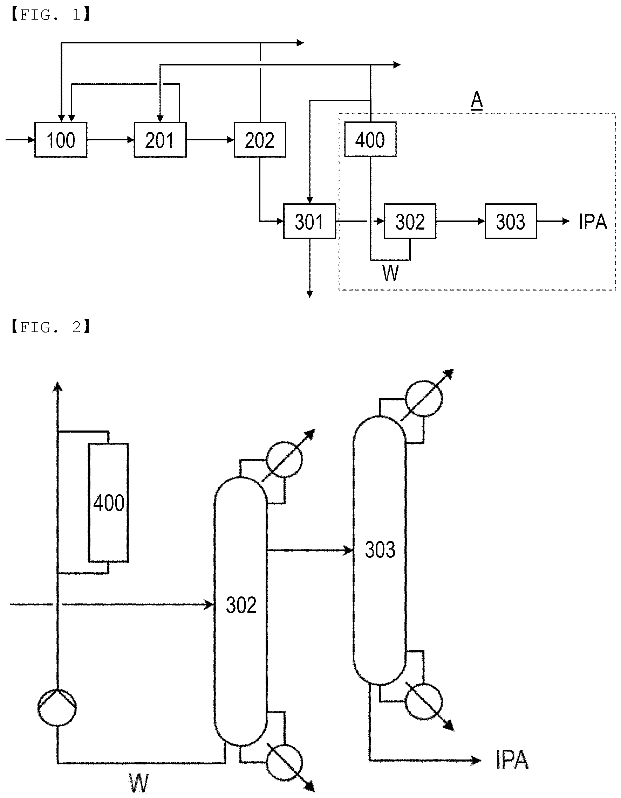

FIG. 1 illustrates the method for preparing isopropyl alcohol according to an exemplary embodiment of the present disclosure, in which isopropyl alcohol may be prepared using a system including a reactor 100, an absorption tower 201, a gas purification part 202, an organic material removal tower 301, a water removal tower 302, an IPA separation part 303, and an arsenic removal device 400.

According to an implementation example, a gaseous reaction product obtained in the reactor 100 passes through the absorption tower 201, the gas purification part 202, the organic material removal tower 301, the water removal tower 302, and the IPA separation part 303 to obtain purified IPA, and water recovered in the lower portion of the water removal tower is used as process water. The process water (W) passes through the arsenic removal device 400, is partly transferred to the absorption tower 201 and the organic material removal tower 301, respectively, and is used as washing water, and the rest is discharged as wastewater.

Usually, water used as a raw material for preparing isopropyl alcohol, that is, reaction water is supplied to a reactor after being heated from an initial temperature of 30°C to 50°C (for example, 40°C) to a target temperature of at least 100°C for a gas phase reaction with a propylene monomer.

Process water (W) may include washing water and unreacted water which is recovered from a process of purifying isopropyl alcohol by separating unreacted propylene monomer, by-products, and the like, while the gaseous reaction product passes through the absorption tower 201, the organic material removal tower 301, the water removal tower 302, and the like which are the rear stage processes.

FIG. 2 shows the part A of FIG. 1 which is a process tower at the rear stage, more specifically. Referring to FIG. 2, process water (W) recovered in a water removal tower 302 passes through the arsenic removal device 400 in order to use for washing a process tower at the front stage (for example, the absorption tower 201 and the organic material removal tower 301), and arsenic may be continuously removed.

The size (volume) of the arsenic removal device 400 may be appropriately designed and changed depending on the flow rate of the process water (W) added to the arsenic removal device, and for example, the empty bed contact time (EBCT) of the process water (W) passing through the arsenic removal device may be 10 minutes to 6 hours, preferably 10 minutes to 4 hours, and more preferably 10 minutes to 1.5 hours. When the arsenic removal device satisfies the empty bed contact time range, arsenic removal efficiency may be improved. In addition, when the arsenic removal efficiency is improved, product production volume may be increased, thereby improving economic feasibility.

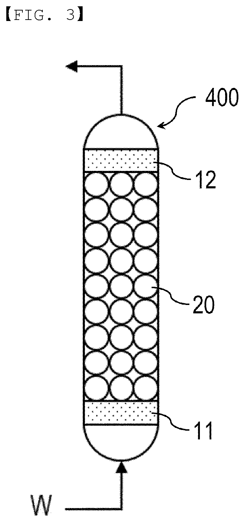

FIG. 3 shows the structure of the arsenic removal device 400 according to an exemplary embodiment. Referring to FIG. 3, the arsenic removal device 400 may have a structure in which the first filtration layer 11, the adsorbent layer 20, and the second filtration layer 12 are sequentially laminated, and may be vertically placed. Accordingly, the process water (W) may be added to one side of the arsenic removal device 400, pass through the first filtration layer 11, the adsorbent layer 20, and the second filtration layer 12 sequentially, and then be discharged to the other side.

The first filtration layer 11 and the second filtration layer 12 are provided in order to suppress elution of the arsenic adsorbent described later and impart a physical filtration effect, and the type may be the same or different. As the first filtration layer 11 and the second filtration layer 12, for example, activated carbon, sand, zeolite, silica gel, metal mesh net, or the like may be used.

In addition, the first filtration layer 11 and the second filtration layer 12 in the arsenic removal device 400 may be independently of each other provided to have the empty bed contact time of 1 to 30 minutes, specifically 1 to 20 minutes, and more specifically 1 to 10 minutes. The elution suppression of the adsorbent and the physical filtration effect may be maximized by satisfying the range.

As illustrated in FIG. 3, the adsorbent layer 20 is provided in order to remove a trace amount of arsenic included in the process water (W) by adsorption and is placed between the first filtration layer 11 and the second filtration layer 12.

The arsenic adsorbent usable as the adsorbent layer 20 may be, for example, a metal oxide-based adsorbent such as iron hydroxide, activated alumina, titanium oxide, α-iron oxide, and γ-iron oxide, an ion exchange resin, and the like. The arsenic adsorbent may have different sizes depending on the shape and type. For example, the adsorbent based on the metal oxide may be in a pellet form or granular form, and the size may be in a range of 0.5 mm to 100 mm, but is not limited thereto. The ion exchange resin may be a gel or microporous type, and the size may be 300 µm to 1,200 µm, but is not limited thereto.

In addition, the adsorbent layer 20 may be provided so as to have the empty bed contact time of 10 minutes to 5 hours, preferably 10 minutes to 3 hours, and more preferably 10 minutes to 1 hour in the arsenic removal device 400. 60 wt% or more, specifically 60 wt% to 99.5 wt%, and more specifically 64 wt% to 99.3 wt% of arsenic in the flow rate of process water (W) passing through the arsenic removal device may be removed by satisfying the range.

According to an exemplary embodiment of the present disclosure, the adsorbent layer 20 may be divided into 1 to n areas, and the first to the nth areas may be filled with first to nth adsorbent, respectively. Herein, each area may be filled with the same or different types of adsorbent, but includes at least one arsenic adsorbent. That is, the types of first to nth adsorbents may be the same as or different from each other. As compared with the structure in which the adsorbent layer is not divided, in the structure in which the adsorbent layer is divided into a plurality of areas, the length of a flow path through which the process water passes the adsorbent layer is increased, and thus, an area and time in and during which the process water comes into contact with the arsenic adsorbent are sufficiently secured. Accordingly, the arsenic adsorbent efficiency may be improved without changing the size of a device.

In addition, when the adsorbent layer 20 having a structure in which the inside of the arsenic removal device is divided into first to nth areas is provided, when the process water (W) passes through the arsenic removal device 400, the process water (W) may be supplied to one side of a first area 1, pass from the first area to the nth area sequentially, and then be discharged in one direction selected from one side and the other side of the nth area. For example, when the inside is divided into the odd number of areas, the process water may be discharged to the other side of the nth area, and when the inside is divided into the even number of areas, the process water may be discharged to one side of the nth area.

In addition, n may be an integer of 2 to 5, preferably 3 or 4. For example, the inside of the arsenic removal device 400 may be divided into 2 or more, preferably 2 to 5, and more preferably 3 or 4 areas by a partition wall 30, and the number of divided areas is preferably an odd number.

Specifically, the partition wall 30 may be provided with a structure which extends radially from the central axis of the arsenic removal device. In addition, the partition wall between two areas may be provided with a flow path in which at least one side is open so that the process water may move between areas adjacent to each other among the first to nth areas. However, though the first area in which the process water inlet exists and the nth area in which the process water outlet exists are adjacent to each other, no open flow path should exist in the partition wall therebetween. More specifically, a flow path open in the other side (C, E) direction is formed in the partition wall between an odd numbered area (for example, the first area) and an even numbered area (for example, the second area), and a flow path open in the other side (B, D) direction may be formed in the partition wall between an even numbered area (for example, the second area) and an odd numbered area (for example, third area). Through the structure, the process water added to the adsorbent layer may come into contact with the first to nth adsorbents in the first to nth areas and then be discharged.

Meanwhile, in order to prevent the arsenic adsorbent from flowing out of the arsenic removal device, among the areas divided by the partition wall, the first filtration layer 11 may be provided in the process water inlet side of the first area through which the process water first passes, and the second filtration layer 12 may be provided in the process water outlet side of the nth area through which the process water passes at the end, but is not limited thereto. The type, shape, size, and empty bed contact time of the adsorbent layer and the filtration layer are as described above.

Furthermore, an inlet (not shown) for adding the adsorbent to the inside and an outlet (not shown) for discharging the adsorbent to the outside may be further provided in the side of the arsenic removal device. Herein, the inlet is placed in the relatively upper portion, and the outlet is placed in the relatively lower portion. When different types of adsorbents are used in each area, lifespan, possibility of reuse, and a reuse method may be different from each other depending on the fluid, and when the inlet and outlet are provided, ad adsorbent inside the arsenic removal device may be exchanged, if necessary.

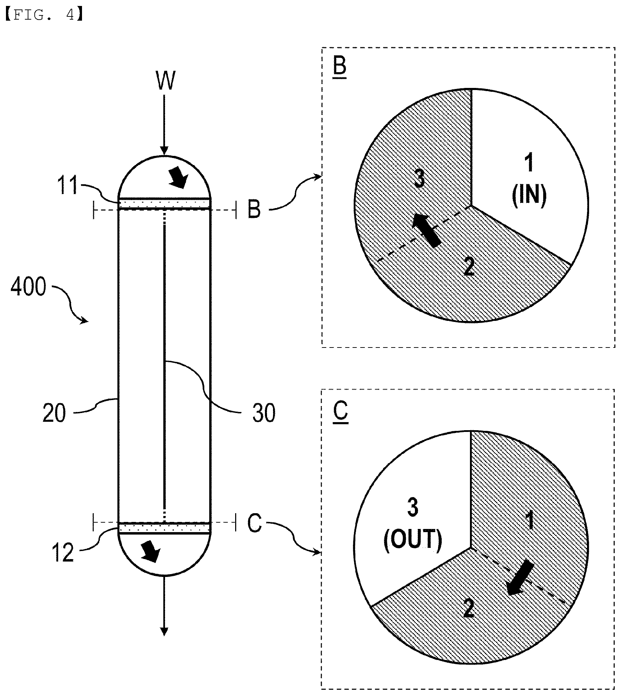

FIG. 4 shows a structure in which the adsorbent layer 20 of the arsenic removal device 400 according to an exemplary embodiment is divided into the first to third areas 1, 2, 3 by partition walls 30a, 30b, 30c; and the right drawing is a longitudinal sectional view of the device, the right upper drawing is a cross sectional view of one side (B) of the device, and the right lower drawing is a cross sectional view of the other side (C) of the device.

As shown in FIG. 4, when the number of inner areas divided by the partition wall 30 is an odd number, the process water (W) may be added to one side of the arsenic removal device, pass through the adsorbent in each area, and then be discharged to the other side of the arsenic removal device, which is thus preferred.

Specifically, referring to FIG. 4, when the inside of the arsenic removal device 400 according to an exemplary embodiment is divided into a first area 1, a second area 2, and a third area 3 by partition walls 30a, 30b, 30c, the process water (W) may be added to one side (B) of the first area 1, pass through the second area 2, and then be discharged to the other side (C) of the third area 3.

More specifically, the device may have a structure in which one side (B) of the partition wall 30b between the second area 2 and the third area 3 is open, and the other side (C) of the partition wall 30a between the first area 1 and the second area 2 is open. In addition, one side (B) of the first area 1 may have a structure having a pipe connected or being open so that the process water (W) may be added thereto, and the other side (C) of the third area 3 may have a structure having a pipe connected or being open so that the process water (W) may be discharged therefrom. The arsenic removal device 400 has the structure as such, thereby moving the process water (W) supplied in the direction of one side (B) to "one side of the first area 1 → the other side of the first area 1 → the other side of the second area 2 → one side of the second area 2 → one side of the third area 3 → the other side of the third area 3" sequentially, and finally discharging the process water in the direction of the other side (C) of the arsenic removal device 400.

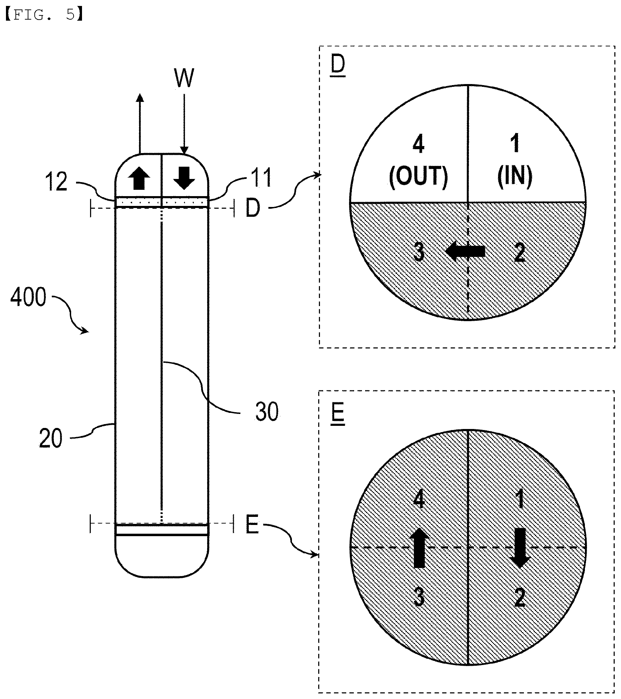

FIG. 5 shows a structure in which the adsorbent layer 20 of the arsenic removal device 400 according to an exemplary embodiment is divided into 4 areas by the partition walls 30a, 30b, 30c, and 30d; and the left drawing is a longitudinal sectional view of the device, the right upper drawing is a cross sectional view of one side (D) of the device, and the right lower drawing is a cross sectional view of the other side (E) of the device.

As shown in FIG. 5, when the number of inner areas divided by the partition wall 30 is an even number, the process water (W) may be added to one side of the arsenic removal device, pass through the arsenic adsorbent in each area, and then be discharged in the same direction as the direction of process water addition.

Specifically, referring to FIG. 5, when the inside of the arsenic removal device 400 according to an exemplary embodiment is divided into a first area 1, a second area 2, a third area 3, and a fourth area 4 by partition walls 30a, 30b, 30c, 30d, the process water (W) may be added to one side of the first area 1, pass through the second area 2 and the third area 3, and then be discharged through the fourth area 4.

More specifically, the device may have a structure in which one side (D) of the partition wall 30b between the second area 2 and the third area 3 is open, and the other side (E) of the partition wall 30a between the first area 1 and the second area 2 and the partition wall 30c between the third area 3 and the fourth area 4 is open. In addition, one side (D) of the first area 1 and the fourth area 4 may independently of each other have a structure having a pipe connected or being open so that the process water (W) may be added or discharged. The arsenic removal device 400 has the structure as such, thereby moving the process water (W) supplied in the direction of one side (D) to "one side of the first area 1 → the other side of the first area 1 → the other side of the second area 2 → one side of the second area 2 → one side of the third area 3 → the other side of the third area 3 → the other side of the fourth area 4 → on side of the fourth area 4" sequentially, and finally discharging the process water in the direction of one side (D) of the arsenic removal device 400.

Meanwhile, the temperature of the process water (W) passing through the arsenic removal device 400 may be 20°C to 150°C or 30°C to 60°C, and the temperature range may be different depending on the type of arsenic adsorbent.

According to an exemplary embodiment of the present disclosure, as illustrated in FIG. 2, when the process water (W) discharged to the lower portion of the water removal tower 302 is passed through the arsenic removal device 400, the process water (W) is branched and only some streams may pass through the arsenic removal device 400. Specifically, when as much process water (W) passes through the arsenic removal device as possible, an arsenic removal rate is improved, but in the case of adding the entire amount of the process water discharged from the water removal tower 302 to the arsenic removal device, the amount may be too much. In this case, considering the size (volume) of the arsenic removal device and/or the economic point of view, it is preferred to appropriately adjust the flow rate of the process water (W) added to the arsenic removal device. For example, the flow rate ratio (v/v) of the process water passing through the arsenic removal device may be 1% or more, 2% or more, or 3% or more and 10% or less, 15% or less, or 20% or less, based on the total volume flow rate ratio of the process water.

In addition, if necessary, two or more arsenic removal devices may be provided in parallel. In this case, the process water (W) discharged to the lower portion of the water removal tower may be branched into two or more and passes through two or more arsenic removal devices to increase the arsenic removal rate in the process water.

Meanwhile, the process water (W) may pass through a heat exchanger (not shown) and be cooled. For example, the cooled process water may be branched and used as the washing water of the absorption tower 201 and the washing water of the organic material removal tower 301. In addition, a part of the cooled process water may be treated as wastewater, and, if necessary, pass through an additional cooler and then be branched, and a part may be used for adjusting the temperature of the washing water and the rest may be discharged as wastewater.

Reaction water is supplied to the reactor 100 and reacts with the propylene monomer in a gas phase to obtain a reaction product including isopropyl alcohol.

Since the propylene monomer used as a raw material is only partly used in the reaction, the reaction product may include 65 wt% to 85 wt% of the unreacted propylene monomer, 4 wt% to 8 wt% of isopropyl alcohol, and 5 wt% to 30 wt% of water. In addition, the reaction product may further include high boiling point organic materials such as isopropyl ether (DIPE) and hexene, and other by-products such as acetone and n-propyl alcohol (NPA). Therefore, a rear stage process for purifying isopropyl alcohol from the reaction product is performed.

First, the gaseous reaction product discharged from the reactor 100 is supplied to an absorption tower 201 and brought into contact with the washing water to obtain an aqueous solution including isopropyl alcohol. As described above, a part of the process water (W) recovered in the water removal tower 302 at the rear stage may be used as the washing water of the absorption tower 201.

Specifically, the reaction product may be supplied to the lower stage of the absorption tower 201, the washing water may be supplied from the upper stage of the absorption tower 201, gaseous isopropyl alcohol is absorbed by the washing water and obtained as a lower liquid stream, and a gaseous stream including the unreacted propylene monomer may be separated in the upper portion and recovered to the reactor 100.

The lower liquid stream of the absorption tower 201 may include the unreacted propylene monomer in a small amount, for example, 5 wt% or less or 2 wt% to 5 wt%, in addition to isopropyl alcohol and unreacted water.

According to an exemplary embodiment of the present disclosure, the volume flow rate of the washing water supplied to the absorption column 201 may be 15 vol% to 40 vol% or 15 vol% to 35 vol% of the flow rate of the reaction product. When the washing water is supplied at the flow rate within the range, an excessive increase in energy expense for recovering the washing water in the rear stage may be prevented, while simultaneously improving absorbency of isopropyl alcohol included in the reaction product.

The absorption tower 201 may be operated at a temperature of 90°C to 100°C or 90°C to 95°C and a pressure of 25 kg/cm² (g) to 40 kg/cm² (g) or 25 kg/cm² (g) to 35 kg/cm² (g). When the operation conditions are satisfied, an upper discharge stream including the unreacted propylene monomer and a lower discharge stream including isopropyl alcohol may be effectively separated.

The liquid stream including isopropyl alcohol separated from the absorption tower 201 may be supplied to a gas purification part 202 and separated into an upper stream including low-boiling point components and a lower stream including isopropyl alcohol, water, and by-products.

The upper stream including low-boiling point components separated in the gas purification part 202 may include an unreacted propylene monomer and inert gas (for example, ethane or propane).

The lower stream separated in the gas purification part 202 may include isopropyl alcohol, water, and by-products (for example, isopropyl ether (DIPE), hexene, n-propyl alcohol (NPA), and the like).

The gas purification part 202 may include one or more flash drums and one or more gas purification towers. First, low-boiling point components are first separated in the flash drum and are supplied to the gas purification tower again to separate the unreacted propylene monomer and the low-boiling point components of inert gas in the upper portion and a small amount of high-boiling point components in the lower portion.

The unreacted propylene monomer separated in the gas purification part 202 may be recovered to the reactor 100, and the inert gas may be branched for exhaust.

Meanwhile, the lower stream of the gas purification part 202 is transferred to an IPA purification part for recovering isopropyl alcohol. The IPA purification part includes an organic material removal tower 301, a water removal tower 302, and an IPA separation part 303.

First, the lower stream from the gas purification part 202 is supplied to the organic material removal tower 301 and brought into contact with washing water in the organic material removal tower 301 to be separated into a liquid phase including isopropyl alcohol and water and a liquid phase including an organic material. As described above, a part of the process water (W) recovered in the water removal tower 302 at the rear stage may be used as the washing water of the organic material removal tower 301.

An alcohol component is dissolved in the washing water, the liquid phase including isopropyl alcohol and water of the organic material removal tower 301 may be separated to the lower portion, and the remaining liquid phase including an organic material may be removed in a layer separator (decanter) at the rear stage of the connected condenser.

In an exemplary embodiment of the present disclosure, the volume flow rate of the washing water supplied to the organic material removal tower 301 may be 60 vol% to 100 vol% or 65 vol% to 95 vol% the flow rate of the lower stream of the gas purification part 202. When the washing water is supplied at the flow rate within the range, an excessive increase in energy expense for recovering the washing water in the rear stage may be prevented, while simultaneously improving absorbency of isopropyl alcohol included in the reaction product.

The liquid phase including isopropyl alcohol and water separated in the organic material removal tower 301 may include 3 wt% to 10 wt% of isopropyl alcohol and 90 wt% to 97 wt% of water. That is, since most of the liquid phase includes washing water, separation is needed.

Therefore, the liquid phase including isopropyl alcohol and water separated in the organic material removal tower 301 is supplied to the water removal tower 302 and separated into an upper stream including isopropyl alcohol and a lower stream including water.

The upper stream separated in the water removal tower 302 includes an azeotropic mixture including isopropyl alcohol and water, and for example, may include 80 wt% to 90 wt% of isopropyl alcohol and 10 wt% to 20 wt% of water.

Meanwhile, the lower stream of the water removal tower 302 is recovered as process water (W) and then passes through the arsenic removal device 400 to remove arsenic, and a part may be recycled as washing water and the rest may be discharged as wastewater.

The water removal tower 302 may be operated at a temperature of 70°C to 150°C or 80°C to 140°C and a pressure of 1 kg/cm² (g) to 5 kg/cm² (g) or 1 kg/cm² (g) to 2 kg/cm² (g). When the operation conditions are satisfied, the azeotropic mixture of isopropyl alcohol and water may be effectively separated.

Thereafter, an azeotropic mixture stream separated in the water removal tower 302 is supplied to an IPA separation part 303 including an IPA purification tower and a solvent recovery tower to recover IPA.

For example, when an organic solvent (for example, cyclohexane, benzene, and the like) is added to the IPA purification tower of the IPA separation part 303 as an azeotropic agent, azeotropy of isopropyl alcohol and water may be damaged to obtain highly purified isopropyl alcohol. In addition, a stream including an azeotropic agent and water may be separated to the upper portion of the IPA purification tower and then supplied to a solvent recovery tower to separate the upper stream of the azeotropic agent and the lower stream of water, and the upper stream of the azeotropic agent may be refluxed to the IPA purification tower.

In the present disclosure, if necessary, devices such as a distillation column, a condenser, a reboiler, a valve, a pump, a separator, and a mixer may be further used.

Hereinabove, the method for preparing isopropyl alcohol according to the present disclosure has been described and illustrated in the drawings, but the description and the illustration in the drawings are the description and the illustration of only core constitutions for understanding of the present disclosure, and in addition to the process and apparatus described above and illustrated in the drawings, the process and the apparatus which are not described and illustrated separately may be appropriately applied and used for carrying out the method for preparing isopropyl alcohol according to the present disclosure.

Hereinafter, the present disclosure will be described in detail through the following examples. However, the following examples are intended to describe the present disclosure in more detail, and the scope of the present disclosure is not limited to the following examples.

### [Examples]

### Example 1

Isopropyl alcohol was produced and purified according to a process system as shown in FIGS. 1 and 2, and at this time, a part of 100 m³/h of process water (W) recovered during the purification process was branched and passed through an arsenic removal device 400 as shown in FIG. 3 to remove arsenic in the process water.

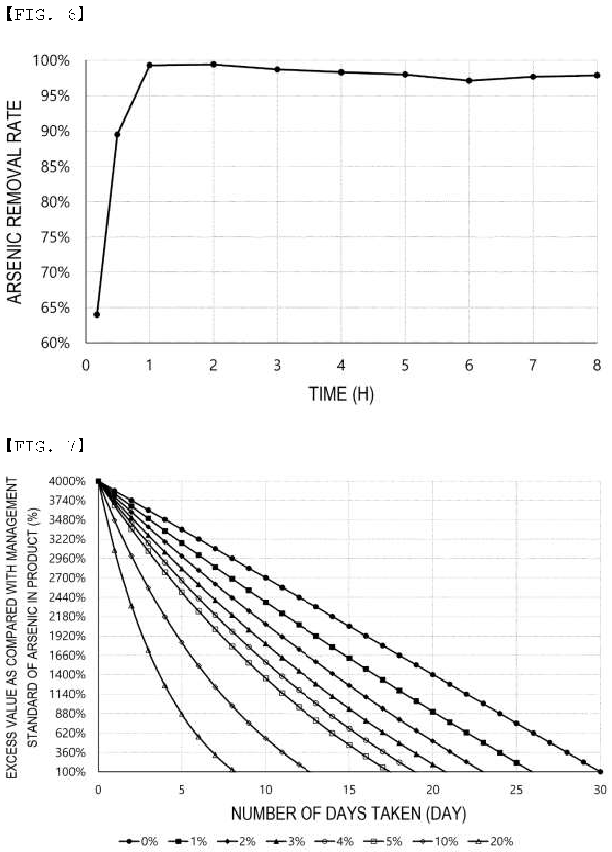

Specifically, as shown in FIG. 3, process water (W) branched at different volume flow rates was passed the arsenic removal device 400 having a volume of 10 m³ having an inner structure in which a first filtration layer 11, an adsorbent layer 20 including an arsenic adsorbent based on iron hydroxide, and a second filtration layer 12 are laminated sequentially, and at this time, an arsenic (As (V)) concentration in the process water before being supplied to the arsenic removal device was 1 ppb (1,000 ppt), and the temperature of the process water was room temperature. In addition, an arsenic concentration in the process water after passing through the arsenic removal device was measured, and at this time, the arsenic removal rate depending on the empty bed contact time (EBCT) of the process water passing through the arsenic removal device is shown in FIG. 6.

Referring to FIG. 6, when the EBCT of the process water passing through the arsenic removal device was 10 minutes (that is, the volume flow rate of the branched process water was 60 m³/h), the arsenic removal rate was about 63 wt%; when the EBCT was 30 minutes (that is, the volume flow rate of the branched process water was 20 m³/g), the arsenic removal rate was about 90 wt%; and when the EBCT was 1 hour or more (that is, the volume flow rate of the branched process water was 10 m³/h), it was found that about 99 wt% or more of arsenic was removed.

### [Experimental Examples]

When arsenic was introduced to the process due to the catalyst exchange in the IPA process, the arsenic concentration in a product began to be detected at a numerical value about 40 times higher than the management standard, and the arsenic concentration tended to decrease over time. Since the flow rate of the process water as in Example 1 was 8 to 9 times higher than the flow rate of pure IPA due to the process characteristics, and circulated and recycled in the process, most of the arsenic concentrated once in the process was concentrated in the process water, only a part was included in IPA and eluted, and thus, when arsenic at a high concentration was added to the process once, it stayed in the process for a long time.

Thus, in the present experimental example, in order to confirm the effect of remove arsenic in the produce depending on whether the arsenic removal process according to the present disclosure was introduced, arsenic was added to the IPA preparation process so that 40 times or more arsenic was detected as compared with the management standard and the change of the arsenic concentration in the IPA product over time was confirmed, and the results are shown in Table 1 and FIG. 7.

In the following Table 1, Comparative Experimental Example 1 did not perform the arsenic removal process according to the present disclosure was not performed, and Experimental Examples 1 to 6 performed the arsenic removal process as in Example 1.

Specifically, in Experimental Examples 1 to 6, a part of the process water was branched and passed through the arsenic removal devices having different sizes, and at this time, the arsenic removal experiment was performed by adjusting the flow rate of the process water to be added to each arsenic removal device with different branching ratios of the process water as shown in the following Table 1 so that the EBCT of each experimental example was 1 hour. In addition, in Experimental Example 7, the arsenic removal experiment was performed under the same conditions as in Experimental Example 6, except that the arsenic removal process was performed using two arsenic removal devices connected in parallel.

In addition, the "number of days taken" in the following Table 1 and FIG. 7 was measurement of a period from the time when arsenic was added until the arsenic concentration in the IPA product was normalized, that is, the number of days it took for the arsenic concentration in the product to reach 100% from 4,000% as compared with the management standard for each arsenic removal rate (wt%) of the process water. In addition, the "number of days shortened" was comparison of a shortened degree of the days taken in Experimental Examples 1 to 7, based on the number of days taken in Comparative Experimental Example 1.

**[Table 1]**

| | Process water branching rate | Arsenic removal rate (%) | Number of days taken (day) | Number of days shortened (day) |
|---|---|---|---|---|
| Comparative Experimental Example 1 | - | 0 | 30 | - |
| Experimental Example 1 | 1 | 1 | 26 | 4 |
| Experimental Example 2 | 2 | 2 | 23 | 7 |
| Experimental Example 3 | 3 | 3 | 21 | 9 |
| Experimental Example 4 | 4 | 4 | 19 | 11 |
| Experimental Example 5 | 5 | 5 | 18 | 12 |
| Experimental Example 6 | 10 | 10 | 13 | 17 |
| Experimental Example 7 | 10/10 | 20 | 9 | 21 |

Referring to Table 1 and FIG. 7, in Comparative Experimental Example 1 in which the arsenic removal process according to the present disclosure was not performed, the arsenic concentration was lowered to 100% from 4,000% over about a month, as compared with the management standard. This means that IPA produced for 30 days did not satisfy the management standard and was inappropriate for IPA used in a semiconductor process.

Meanwhile, in Experimental Examples 1 to 7 to which the arsenic removal process according to the present disclosure was introduced was able to shorten the period from the time when the arsenic was added until normalization, by removing arsenic in the process water. In addition, as the arsenic removal rate in the process water was higher, the number of days it took for the arsenic concentration in the product to be normalized was further shortened, and a high-quality product production amount was able to be secured by the shortened number of days, which was advantageous from an economic perspective.

In addition, in Experimental Example 7 in which two arsenic removal devices were connected in parallel to perform the arsenic removal process, the number of days taken for the arsenic concentration in the product to normalize was not greatly shortened as compared with Experimental Example 6 using one arsenic removal device. Considering these facts, it is considered that the process needs to be appropriately designed after comparing costs incurred when adding the arsenic removal device and profits which may be obtained from the product production amount increased by the number of days shortened when adding the device.

Hereinabove, the exemplary embodiments of the present disclosure have been described, but the present disclosure is not limited thereto, and those with ordinary skill in the art will understand that various changes and modification are possible within the range which is not out of the concept and the scope of the claims described later.

### [Reference signs list]

100: Reactor
201: Absorption tower
202: Gas purification part
301: Organic material removal tower
302: Water removal tower
303: IPA separation part
400: Arsenic removal device
11, 12: Filtration layer
5 20: Adsorbent layer
30, 30a, 30b, 30c: Partition wall

## Claims

1. A method for preparing isopropyl alcohol, the method comprising:
(S1) supplying a propylene monomer and reaction water to a reactor as a feed stream to produce a gaseous reaction product including isopropyl alcohol;
(S2) purifying isopropyl alcohol from the gaseous reaction product and recovering process water; and
(S3) passing the process water through an arsenic removal device including a first filtration layer, an adsorbent layer, and a second filtration layer,
wherein (S3) includes passing the process water through the first filtration layer, the adsorbent layer, and the second filtration layer inside the arsenic removal device sequentially.

2. The method for preparing isopropyl alcohol of claim 1, wherein the first filtration layer, the adsorbent layer, and the second filtration layer are laminated sequentially in the arsenic removal device.

3. The method for preparing isopropyl alcohol of claim 1, wherein the adsorbent layer is divided into first to nth areas, and the first to nth areas are filled with first to nth arsenic adsorbents, respectively.

4. The method for preparing isopropyl alcohol of claim 3, wherein when the process water passes through the adsorbent layer, the process water is supplied to one side of the first area, passes from the first area to the nth area sequentially, and then is discharged in one direction selected from one side and the other side of the nth area.

5. The method for preparing isopropyl alcohol of claim 3, wherein n is an integer of 2 to 5.

6. The method for preparing isopropyl alcohol of claim 1, wherein the adsorbent layer includes one or more arsenic adsorbents selected from iron hydroxide, activated alumina, titanium oxide, α-iron oxide, γ-iron oxide, and an ion exchange resin.

7. The method for preparing isopropyl alcohol of claim 1, wherein the first filtration layer and the second filtration layer independently of each other include one or more selected from activated carbon, sand, zeolite, silica gel, and mesh net.

8. The method for preparing isopropyl alcohol of claim 1, wherein an empty bed contact time of the process water passing through the arsenic removal device is 10 minutes to 6 hours.

9. The method for preparing isopropyl alcohol of claim 1, wherein (S3) includes branching the process water and then passing a part of the process water through the arsenic removal device.

10. The method for preparing isopropyl alcohol of claim 9, wherein a flow rate ratio (v/v) of the process water passing through the arsenic removal device is 1% to 20%, based on the total volume flow rate of the process water.

11. The method for preparing isopropyl alcohol of claim 1, wherein a temperature of the process water is 20°C to 150°C.

12. The method for preparing isopropyl alcohol of claim 1, wherein the gaseous reaction product includes isopropyl alcohol, unreacted propylene, unreacted water, and by-products.

13. The method for preparing isopropyl alcohol of claim 1, wherein (S2) includes:
(i) supplying the gaseous reaction product to an absorption tower and bringing the gaseous reaction product into contact with washing water to obtain an aqueous solution including isopropyl alcohol,
(ii) supplying the aqueous solution including isopropyl alcohol to a gas purification part and separating an upper stream including low-boiling point components and a lower stream including isopropyl alcohol, water, and by-products,
(iii) supplying the lower stream of the gas purification part to an organic material removal tower and bringing the stream into contact with washing water to separate a liquid phase including isopropyl alcohol and water and a liquid phase including an organic material, and
(iv) supplying the liquid phase including isopropyl alcohol and water to a water removal tower to separate an upper stream including isopropyl alcohol and a lower stream of water, purifying isopropyl alcohol from the upper stream, and recovering the lower stream as the process water.

14. The method for preparing isopropyl alcohol of claim 13, wherein the washing water used in the absorption tower and the organic material removal tower is the process water recovered in (S2).
